**Europäisches Patentamt**

**European Patent Office**

**Office européen des brevets**

(19)

(11) Veröffentlichungsnummer: **0 304 852 B1**

# EUROPÄISCHE PATENTSCHRIFT

(12)

(45) Veröffentlichungstag der Patentschrift:
**16.10.91 Patentblatt 91/42**

(51) Int. Cl.$^5$: **B01J 29/28**

(21) Anmeldenummer: **88113655.0**

(22) Anmeldetag: **23.08.88**

(54) **Alkalifreie Herstellung von grossen Pentasil-Kristallen als Schalen- oder Vollkatalysatoren.**

(30) Priorität: **26.08.87 DE 3728451**

(43) Veröffentlichungstag der Anmeldung:
**01.03.89 Patentblatt 89/09**

(45) Bekanntmachung des Hinweises auf die
Patenterteilung:
**16.10.91 Patentblatt 91/42**

(84) Benannte Vertragsstaaten:
**BE DE FR GB IT NL**

(56) Entgegenhaltungen:
**EP-A- 0 014 546**
**EP-A- 0 041 621**
**EP-A- 0 178 687**
**EP-A- 0 219 804**

(56) Entgegenhaltungen:
**PROCEEDINGS OF THE 7TH INTERNATIONAL
ZEOLITE CONFERENCE TOKYO, 17.-22. August 1986, Seiten 121-128, Herausgeber Y. MURAKAMI et al., Elsevier, Amsterdam, NL; J.L.
GUTH et al.: "New route to pentasil-type zeolites using a non alkaline medium in the presence of fluoride ions"**

(73) Patentinhaber: **BASF Aktiengesellschaft
Carl-Bosch-Strasse 38
W-6700 Ludwigshafen (DE)**

(72) Erfinder: **Danner, Alfred
Hochzeitsweg 11
W-6140 Bensheim (DE)**
Erfinder: **Mueller, Ulrich
Neustrasse 3
W-6229 Schlangenbad 2 (DE)**
Erfinder: **Unger, Klaus, Prof. Dr.
Am Alten Berg
W-6104 Seeheim (DE)**
Erfinder: **Hoelderich, Wolfgang, Dr.
Mannheimer Strasse 18 c
W-6710 Frankenthal (DE)**

EP 0 304 852 B1

## Beschreibung

Bei der Herstellung von Formkörpern aus Zeolith-Pulver ist die Verwendung von Bindern unerläßlich. Als Binder kommen organische Verbindungen wie Ethyl-Cellulose (US 4333768) oder Polykieselsäureester (DE 3231498) wie auch anorganische Verbindungen wie Aluminiumoxid (US 4563435), Silica-Gel (EP 167324) oder Tone (DE 3208672) in Frage.

Hydrothermale Umsetzungen von vorgeformten Trägern aus tonhaltigen Materialien, teilweise mit entsprechendem Zeolithzuschlag zu Zeolithformkörper sind z.B. bekannt für Mordenit (US 3445184), Faujasit (DE 1567894) oder Zeolith A (DE 3242126), wobei jeweils Pellets entstehen, die zu 100% aus den entsprechenden Zeolithen bestehen. Auch die Herstellung von Zeolithen vom Typ ZSM-5 ist auf diese Weise bekannt (EP 201264), wobei die Verwendung von Impfkristallen Voraussetzung ist und die Umsetzung zu ZSM-5 nur mit schlechten Ausbeuten erfolgt. Zeolithe vom Typ ZSM-5 sind in US 3702886 beschrieben, ebenso ihre Herstellung in Pulverform.

EP-A-0041621 offenbart eine Methode zur Kristallisierung von Pentasil-Kristallen aus alkalifreien Mischungen, die Kieselsäure in Pulverform enthalten.

Es wurde nun gefunden, daß man bei der alkalifreien Herstellung von großen Pentasil-Kristallen als Schalen- und Vollkatalysatoren, bei denen die einzelnen Pentasil-Kristalle größer als 5 µm sind, durch hydrothermale Behandlung von $SiO_2$ gute Ergebnisse erzielt, wenn man $SiO_2$-Formkörper einer hydrothermalen Behandlung bei 140 bis 220°C in einer alkalifreien Mischung aus Wasser, einem Amin und/oder Tetraalkylammoniumverbindung und zusätzlich gegebenenfalls Ammoniak unterzieht.

Bei der erfindungsgemäßen Herstellung von Pentasil-Schalen- und -Vollkatalysatoren durch hydrothermale Umsetzung von $SiO_2$-Pellets in Form von Kugeln oder Extrudaten, geht man von Synthesemischungen aus, die keine Alkalimetallionen enthalten. Die Aluminiumkomponente kann entweder der Reaktionslösung als lösliche Aluminiumverbindung, z.B. als Aluminiumsalz, Aluminiumalkoholat oder Aluminiumhydroxid, zugegeben oder als feste Komponente bereits bei der Herstellung der $SiO_2$-Formkörper eingearbeitet werden, z.B. als Aluminiumoxid oder Pseudoboehmit eingeknetet werden.

Als $SiO_2$-Quelle für die Herstellung der Formkörper kommen alle gängigen $SiO_2$-haltigen Materialien, z.B. Silica-Gel oder pyrogene Kieselsäure oder hochdisperses Siliciumdioxid, in Frage. Das $SiO_2$-$Al_2O_3$-Verhältnis kann von ∞ bis 8 : 1 variiert werden. Bevorzugt ist ein Verhältnis von 100 : 1 bis 30 : 1.

Durch das erfindungsgemäße Verfahren werden in den Formkörpern Pentasil-Kristalle gebildet, die in der Längsachse vermessen in ihrer Masse größer als 5 µm sind. Durch Einstellung der Verfahrensbedingungen z.B. Synthesdauer, $H_2O$/$SiO_2$-Verhältnis kann man auch Größen von 50 bis 200 µm erzielen. Kristalle dieser Größe zeichnen sich durch eine hohe Form-Selektivität aus. Wird die Aluminiumkomponente bei der Formkörperherstellung eingearbeitet, so kann dies als Pulver geschehen oder als mit einer Mineralsäure, z.B. Salpetersäure, peptisiertem Gel, wobei der Säureanteil von 0,016 bis 0,227 g/g $Al_2O_3$ variiert werden kann. Bei Zusatz von Mineralsäure sinkt die Kristallgröße ab.

Als Binderflüssigkeit können Ammoniak, Amine und Wasser verwendet werden. Bevorzugt wird verdünnte Ammoniaklösung verwendet. Die Menge des Ammoniakanteils kann von 0 bis 32%, bevorzugt 2,5% betragen. Die Menge der Binderflüssigkeit kann von 125 bis 170 g/100 g $SiO_2$, bevorzugt 150 bis 160 g/100 g $SiO_2$ betragen. Bei der Formgebung kann man bekannte Verfahren, z.B. die Extrusion, anwenden. Die erhaltenen Formkörper können im getrockneten wie auch im calcinierten oder teilweise gesinterten Zustand umgesetzt werden.

Zur Umwandlung der bei etwa 100 bis 140°C getrockneten und/oder bei 500 bis 600°C calcinierten amorphen Formkörper zu Pentasilzeolith, werden diese mit einer wäßrigen Lösung aus einem Amin oder einer Tetraalkylammoniumverbindung, bevorzugt ein Tetra-n-propylammoniumsalz oder -hydroxid, und gegebenenfalls in Wasser gelöstem $NH_3$ getränkt, und im Autoklaven bei Temperaturen von 140 bis 220°C, bevorzugt 160 bis 200°C, und einer Reaktionsdauer von 1 bis 30 Tagen, bevorzugt 1 bis 7 Tage, hydrothermal umgesetzt. Das Aluminium ist dabei in Form von Pseudoboehmit bereits in den Formkörpern vorhanden.

**Tabelle 1: Reaktionsbedingungen für die hydrothermale Herstellung von Pentasil-Formkörpern**

|  | allgemein | bevorzugt |
|---|---|---|
| $SiO_2/Al_2O_3$ | 8 - ∞ | 30 - 100 |
| $H_2O/SiO_2$ | 5 - 100 | 10 - 40 |
| $OH/SiO_2$ | 0,01 - 6 | 0,13 - 4 |
| $R/SiO_2$ | 0,01 - 0,3 | 0,04 - 0,17 |
| Reaktionszeit | 1 - 30 Tage | 1 - 7 Tage |
| Reaktionstemp. | 140 bis 220°C | 160 bis 200°C |

R = Amin, Tetraalkylammoniumverbindung

Man erhält so Formkörper die durchkristallisiert sind und einen ZSM-5-Gehalt bis zu 95% und Kristallgrößen bis zu 100 µm aufweisen.

Nimmt man z.B. die bei etwa 120°C getrockneten Pellets aus reinem $SiO_2$ und gibt das Aluminium in Form einer löslichen Komponente der oben beschriebenen Syntheselösung zu, so erhält man Formkörper mit einem Kern aus 100% ZSM-5 und einer Schale aus amorphem $SiO_2$. Die Reaktionsbedingungen können Tabelle 1 entnommen werden.

Wenn man bei dem Verfahren von teilweise gesinterten $SiO_2$-Formkörpern (z.B. kommerzielle $SiO_2$-Träger) ausgeht, so bildet sich nur eine aus 1 bis 2 Kristallagen bestehende Schale aus ZSM-5-Kristallen.

Ausschlaggebend dafür, ob ein vollkristalliner Körper entsteht oder ob nur eine Schale entsteht, ist, ob die Ausgangsformkörper eine breite Porenverteilung mit einem oder mehreren breiten Maxima im Bereich um 60 nm Porendurchmesser (vollkristallin), oder eine enge Verteilung mit einem scharfen Maximum bei etwa 8 nm Porendurchmesser (Schale) aufweisen.

Durch 4- bis 30stündiges, bevorzugt 24stündiges, Calcinieren bei 450 bis 600°C, bevorzugt 550°C, werden die Formkörper direkt in die katalytisch aktive H-Form überführt. Die Pentasil-Formkörper zeichnen sich durch eine hohe Formselektivität aus. Bei der Testreaktion für die Formselektivität der synthetisierten Katalysatoren, der Ethylbenzol-Disproportionierung (Karge, Journal of Catalysis 82, S. 236 (1983)), findet man bis zu 96% para-Diethylbenzol im Isomerengemisch der Diethylbenzole.

Der kristalline Pentasil-Anteil in den Formkörpern, im folgenden als Kristallinität bezeichnet, wird durch Behandlung der Formkörper mit etwa 30%iger NaOH bei Raumtemperatur über 5 Tage bestimmt. Die amorphen Anteile werden hierbei herausgelöst. Die Größe der Pentasil-Kristalle wird mit dem Lichtmikroskop und rasterelektronenmikroskopisch bestimmt.

Die erfindungsgemäßen Katalysatoren sind geeignet für Kohlenwasserstoffumwandlungen, z.B. für die Umwandlung von Methanol und/oder Dimethylether zu Kohlenwasserstoffen, die Umsetzung von Epoxiden zu Aldehyden, Aldehyden in Ketone, Skelett- und Valenzisomerisierungen von Kohlenwasserstoffen und die Aminierung von Olefinen, die Hydroformylierung von Olefinen, Dehydratisierungsreaktionen an Alkoholen, Aldehyden und Säureamiden, Acetalspaltungen, Carbonylierungs- und Decarbonylierungsreaktionen, Trägermaterial für Hydrier- und Dehydrierkatalysatoren sowie weitere organische Synthesen (W. Hölderich, Pure and Appl. Chem. 58, 10 (1986) 1383). Insbesondere sind die erfindungsgemäßen Katalysatoren geeignet für Umsetzungen in der Wirbelschicht.

Beispiel 1 bis 9

In den Beispielen 1 bis 6 werden Pentasil-Voll-Katalysatoren aus $SiO_2$-Formkörpern, in die die Aluminiumkomponente eingeknetet wurde, hergestellt.

Beispiel 1

6,6 g Pseudoboehmit (AlOOH, 12% Trocknungsverlust bei 120°C) werden in 200 g 2,5%iger Ammoniaklösung dispergiert, in einen Kneter überführt und 120 g pyrogene Kieselsäure innerhalb von 22 min eingeknetet. Die resultierende Paste wird extrudiert und bei 120°C 24 h im Trockenschrank getrocknet.

12,6 g der getrockneten Extrudate werden zusammen mit einer Lösung aus 7,1 g Tetra-n-propylammoniumbromid, 21,4 g $H_2O$ und 18,1 g 25%iger Ammoniaklösung in einen Autoklaven gegeben und 5 Tage bei 185°C reagieren lassen.

Ein Teil der resultierenden Formkörper wird nach dem Trocknen zur Bestimmung der Kristallinität mit ca. 30%iger NaOH behandelt, wobei sich die amorphen Anteile herauslösen. Ein anderer Teil wird durch 24stündiges Calcinieren bei 550°C in die katalytisch aktive H-Form überführt und anhand der Ethylbenzol-Disproportionierung auf seine Selektivität untersucht. Bei diesem Test war die Reaktionstemperatur stets 250°C und die Sättigertemperatur 20°C. Die Belastung wird so eingestellt, daß ein Umsatz von 2% erreicht wird.

Die resultierenden Extrudate bestehen zu 89% aus NH₄-ZSM-5. Das bei dem katalytischen Test bei 2% Umsatz entstehende Isomerengemisch der 3 Diethylbenzole besteht zu 95,9% aus dem para-Isomeren und 4,1% aus dem meta-Isomeren.

Beispiel 2

Die in Beispiel 1 hergestellten Formkörper werden unter sonst gleichen Bedingungen 1 Tag hydrothermal behandelt. Sie bestehen danach zu 50% aus NH₄-ZSM-5. Der Selektivitätstest ergibt 94,8% para-Diethylbenzol.

Beispiel 3

Die nach Beispiel 1 hergestellten Extrudate werden vor der hydrothermalen Behandlung 4 h bei 550°C calciniert. Unter sonst gleichen Bedingungen entstehen Extrudate mit einem Anteil von 62% NH₄-ZSM-5. Die Selektivität bezüglich des para-Diethylbenzols beträgt 94,7%.

Um den Einfluß der sauren Vorbehandlung der Aluminiumkomponente zu verdeutlichen, dienen die nächsten drei Beispiele.

Beispiel 4

3,3 g Pseudoboehmit (AlOOH, 12% Trocknungsverlust bei 120°C) werden in 150 g 1%iger Ammoniaklösung dispergiert, in einen Kneter überführt und 120 g pyrogene Kieselsäure innerhalb von 22 min eingeknetet. Die Paste wird extrudiert und 12,3 g der wie unter 1. getrockneten Extrudate wie in Beispiel 1 beschrieben hydrothermal umgesetzt.

Die resultierenden Formkörper bestehen zu 83% aus NH₄-ZSM-5 und zeigen eine para-Selektivität von 44,3%.

Beispiel 5

Ein Gel, bestehend aus 3,3 g Pseudoboehmit (AlOOH, 12% Trocknungsverlust bei 120°C) und 4,4 g 1%iger Salpetersäure, wird in 145,4 g 1%iger Ammoniaklösung dispergiert, mit 120 g pyrogener Kieselsäure verknetet, extrudiert und wie in Beispiel 4 weiterbehandelt, aber nur 2 Tage hydrothermal umgesetzt. Die resultierenden Formkörper zeigen eine Kristallinität von 38% und eine para-Selektivität von 54,6% für Diethylbenzol.

Beispiel 6

3,3 g Pseudoboehmit (AlOOH, 12% Trocknungsverlust bei 120°C) werden in 150 g 0,5%iger Salpetersäure dispergiert und mit 120 g pyrogener Kieselsäure verknetet. Die weitere Behandlung geschieht wie in Beispiel 4 beschrieben. Die resultierenden Formkörper sind zu 77% kristallin und zeigen eine para-Selektivität von 93,5% für Diethylbenzol.

In den bisher genannten Beispielen lag eine homogene Verteilung der Kristalle über den gesamten Querschnitt der Formkörper vor. Die nächsten Beispiele beschreiben die Ergebnisse bei der Zugabe der Aluminiumkomponente zur Syntheselösung.

Beispiel 7

120 g pyrogene Kieselsäure werden in 200 g 2,5%iger Ammoniaklösung eingeknetet und danach extrudiert. 6,4 g der bei 120°C über 24 h getrockneten Extrudate werden mit einer Lösung aus 3,77 g Tetra-n-propylammoniumbromid, 47,7 g H₂O, 23 g 32%iger Ammoniaklösung und 0,732 g Aluminiumtriisopropylat versetzt und 4 Tage bei 185°C im Autoklaven umgesetzt. Es entstehen Formkörper mit einem Kern, der zu 100% aus NH₄-ZSM-5 besteht und einer Schale aus amorphem SiO₂, die sich beim Calcinieren ablöst. Der katalytische Test, der wie in Beispiel 1 aktivierten Formkörper, erbringt einen Anteil von 94,3% para-Diethylbenzol am Isomerengemisch.

Beispiel 8

100 g kommerziell erhältliche $SiO_2$-Kugeln mit einem Durchmesser von 3 bis 5 mm werden mit einer Lösung aus 59,4 g Tetra-n-propylammoniumbromid, 255 g $H_2O$ und 160 g 25%iger Ammoniaklösung versetzt und im Autoklaven über einen Zeitraum von 5 Tagen bei 185°C umgesetzt. Die erhaltenen Formkörper bestehen aus Kugeln aus amorphem $SiO_2$, auf die eine Schicht aus $NH_4$-ZSM-5 aufgewachsen ist. Eine katalytische Aktivität ist bei diesem aluminiumfrei hergestellten ZSM-5 nicht feststellbar.

Beispiel 9

20 g kommerziell erhältliche $SiO_2$-Kugeln ($\phi$ 1 mm, Kali-Chemie) werden mit einer Lösung aus 11,8 g Tetra-n-propylammoniumbromid, 60,8 g $H_2O$, 29,6 g 25%iger Ammoniaklösung und 0,28 g Pseudoboehmit (AlOOH, 12% Trocknungsverlust bei 120°C) versetzt und im Autoklaven über einen Zeitraum von 5 Tagen bei 185°C umgesetzt. Es entstehen Formkörper aus amorphem $SiO_2$ mit einer aufgewachsenen Schicht aus $NH_4$-ZSM-5. Nach 24stündigem Calcinieren bei 550°C zeigt sie eine para-Selektivität von 94,1%.

In Tabelle 2 sind die Eigenschaften der Formkörper zusammengestellt.

Tabelle 2

| Beispiel Nr. | Kristallinität [%] | Größe der Pentasil-Kristalle [μm] | para-Selektivität im EBD-Test* [%] |
|---|---|---|---|
| 1 | 89 | 90 - 100 | 95,9 |
| 2 | 50 | 50 - 60 | 94,8 |
| 3 | 62 | 95 - 105 | 94,7 |
| 4 | 83 | 7 - 12 | 44,3 |
| 5 | 38 | 6 - 9 | 54,6 |
| 6 | 77 | 7 - 12 | 93,5 |
| 7 | 100 (im Kern) | 90 - 100 | 94,3 |
| 8 | 100 (in Schale) | 50 - 60 | - |
| 9 | 100 (in Schale) | 40 - 50 | 94,1 |

\* EBD = Ethylbenzol-Disproportionierung

## Patentansprüche

1. Verfahren zur alkalifreien Herstellung von großen Pentasil-Kristallen als Schalen- oder Vollkatalysatoren, bei denen die einzelnen Pentasil-Kristalle größer als 5 μm sind, durch hydrothermale Behandlung von $SiO_2$, dadurch gekennzeichnet, daß man die $SiO_2$-Formkörper einer hydrothermalen Behandlung bei 140 bis 220°C in einer alkalifreien Mischung aus Wasser, einem Amin und/oder Tetraalkylammoniumverbindung und zusätzlich gegebenenfalls Ammoniak unterzieht.

2. Verfahren nach Anspruch 1, dadurch gekennzeichnet, daß die Aluminiumkomponente zur Herstellung von Aluminosilikat-Zeolith in den $SiO_2$-Formkörper eingeknetet wird.

3. Verfahren nach Anspruch 1, dadurch gekennzeichnet, daß die Aluminiumkomponente zur Herstellung von Aluminosilikat-Zeolith über die flüssige Phase während der hydrothermalen Behandlung zugeführt wird.

4. Verfahren nach Anspruch 1, dadurch gekennzeichnet, daß man als Tetraalkylammoniumverbindung ein Tetra-n-propylammoniumsalz oder -hydroxid verwendet.

5. Verwendung der nach den Ansprüchen 1 bis 4 hergestellten Schalen- und Vollkatalysatoren für Umsetzungen in der Wirbelschicht.

## Claims

1. A process for the alkali-free preparation of large pentasil crystals as coated catalysts or fully crystalline catalysts, in which the individual pentasil crystals are larger than 5 μm, by hydrothermal treatment of $SiO_2$, wherein the $SiO_2$ moldings are subjected to a hydrothermal treatment at from 140 to 220°C in an alkalifree mixture of water, an amine and/or a tetraalkylammonium compound, with or without ammonia.

2. A process as claimed in claim 1, wherein the aluminum component for the preparation of an aluminosilicate zeolite is kneaded into the $SiO_2$ moldings.

3. A process as claimed in claim 1, wherein the aluminum component for the preparation of an aluminosilicate zeolite is fed in via the liquid phase during the hydrothermal treatment.

4. A process as claimed in claim 1, wherein the tetraalkylammonium compound used is a tetra-n-propylammonium salt or hydroxide.

5. Use of a coated catalyst or fully crystalline catalyst prepared as claimed in claim 1 or 2 or 3 or 4 for a reaction in a fluidized bed.


## Revendications

1. Procédé de préparation, en l'absence de substances alcalines, de gros cristaux de pentasil utilisables comme catalyseurs en coquille ou pleins, les cristaux individuels de pentasil ayant une taille supérieure à 5 μm, par traitement hydrothermal de $SiO_2$, caractérisé en ce qu'on soumet les corps moulés de $SiO_2$ à un traitement hydrothermal à une température de 140 à 220°C dans un mélange exempt de substances alcalines, composé d'eau, d'une amine et/ou d'un composé de tétraalkylammoniun et en outre, le cas échéant, d'ammoniac.

2. Procédé selon la revendication 1, caractérisé en ce que pour la préparation de zéolithe d'aluminosilicate, la composant à base d'aluminiun est incorporé par malaxage dans les corps moulés de $SiO_2$.

3. Procédé selon la revendication 1, caractérisé en ce que pour la préparation de zéolithe d'aluminosilicate, le composant à base d'aluminium est introduit au moyen de la phase liquide pendant le traitement hydrothermal.

4. Procédé selon la revendication 1, caractérisé en ce qu'on utilise, comme composé de tétraalkylammonium, un sel ou un hydroxyde de tétra-n-propylanmonium.

5. Utilisation des catalyseurs en coquille ou pleins préparés selon l'une quelconque des revendications 1 à 4 pour des réactions en lit fluidisé.